# EUROPEAN PATENT SPECIFICATION

(11) **EP 3 684 746 B1**
(45) Date of publication and mention of the grant of the patent: **02.08.2023**
(21) Application number: 17925553.4
(22) Date of filing: 21.09.2017
(51) Int. Cl.: C07C 67/08, C07C 67/54, C07C 67/60, C07C 69/78

(54) **METHOD FOR PREPARING BENZOIC ACID ESTERS**
VERFAHREN ZUR HERSTELLUNG VON BENZOESÄUREESTERN
PROCÉDÉ DE PRÉPARATION D'ESTERS D'ACIDE BENZOÏQUE

(43) Date of publication of application: 29.07.2020
(73) Proprietor: Public Joint Stock Company "Sibur Holding", Tobolsk, Tyumen region 626150 (RU)
(72) Inventor: NOSIKOV, Aleksei Aleksandrovich, s. Turuntaevo 634534 (RU); BABIN, Ivan Anatolievich, g. Tomsk 634031 (RU); POPOVTSEV, Egor Evgenievich, Tomskaya oblast Seversk 636000 (RU); IGASHEVA, Varvara Petrovna, s. Porotnikovo 636213 (RU)
(74) Representative: Peters, Andreas
(86) International application number: PCT/RU2017/000692
(87) International publication number: WO 2019/059801

(56) References cited:
- US-A- 5 087 730
- US-A1- 2007 027 240
- US-A1- 2007 060 768
- US-A1- 2016 264 509
- US-A1- 2016 264 509
- US-B1- 6 235 924
- KESHE MOHAMMAD et al.: "Studying the Optimum Reaction Conditions for Organic Esterification of Glycerol to Benzoic Acid and Some Devices by Using Ecofriendly Media", Chemistry and Materials Research, vol. 8, no. 1, 2016, pages 48-54, XP055584954,

## Description

The invention relates to the field of organic synthesis, in particular, to a field of preparing benzoic acid esters by esterification of said acid and monohydric aliphatic or polyhydric alcohols, selected from the group comprising monohydric alcohols having a number of carbon atoms from 6 to 12, or polyhydric alcohols having a number of carbon atoms from 2 to 10, in the presence of metal containing catalyst and a solvent based on an aromatic compound. The esters obtained according to the invention can be used in paints, inks or lacquers, in plastisols, adhesives or adhesive components, sealing compositions, as well as plasticizers in plastics, or in the plastic components, in particular, as plasticizers for polyvinyl chloride (PVC).

### BACKGROUND

To the benzoic acid esters relate, in particular, monobenzoic acid esters, such as isodecyl benzoate, isononyl benzoate, 2-ethylhexyl benzoate, dipropylen glycol monobenzoate, diethylene glycol monobenzoate, as well as dibenzoate esters, such as diethylene glycol dibenzoate, dipropylene glycol dibenzoate, etc., which are widely used in paints, inks or lacquers, in plastisols, adhesives or adhesive components, sealing compositions, as well as plasticizers in plastics, or in the plastic components, in particular, as plasticizers for polyvinyl chloride (PVC).

Well known are the methods of preparing said esters by esterification of benzoic acid and monohydric and polyhydric alcohols in the presence of esterification catalyst, with subsequent purification of the obtained crude ester, the purification comprising neutralization of acid components, treatment with adsorbents, filtration, providing the recovery of the desired product. Thus, for example, from the prior art known are the methods for producing esters using acid catalysts. The patent CN103739494 discloses a method for producing benzoic acid and dihydric alcohol esters: glycol, in particular, triethylene glycol using p-toluene sulphonic acid as a catalyst. The ratio between benzoic acid and triethylene glycol is 2.1:1. Esterification is conducted at the temperature 170-210°C. After the reaction is completed, the reaction mass is cooled to 70°C and is neutralized by subsequent washing with aqueous solution of sodium hydroxide. The disadvantage of this method is the use of a large amount of a catalyst (0.6% by weight) and a low yield of the desired product (97% by weight). Moreover, the use of the acid catalyst leads to the corrosion of the steel equipment employed in the process of esterification.

From the prior art known is the use of metal containing catalysts in the production of esters, in particular, the titanium compounds. For example, in the patent CN103086891 diethylene glycol and benzoic acid ester is obtained in the presence of butyl titanate (IV) catalyst. The reaction is conducted under vacuum in a glass flask at the molar ration of an acid to glycol of 2:(1.05-1.2) and at the temperature 160-220°C in the presence of 0.6% by weight of a catalyst. The reaction lasts for 3-4 hours with a conversion of glycol up to 99.3%. After the reaction is completed the reaction mass is subsequently washed with aqueous solution of sodium carbonate and pure water. The product is elutriated and is subjected to the vacuum distillation.

The disadvantage of said method is the use of vacuum during the whole process of esterification and a large amount of a catalyst (0.6% by weight).

The patent CN102816068 discloses a method of production of diethylene glycol dibenzoate esters by esterification, wherein as the starting compounds the use is made of benzoic acid and diethylene glycol. As a catalyst system the use is made of a catalyst which is a mixture of titanium (IV) isoproxide and a solid compound of titanium. The esterification process is conducted for 4-6 hours at the temperature 190-210°C at the molar ratio benzoic acid:diethylene glycol of (1.7-1.9): 1. The catalyst amount is 0.05-0.08% by weight. After the reaction is completed the unreacted diethylene glycol is removed by vacuum distillation (dealcolization), and the ester obtained by the described method is refined by activated carbon and/or diatomite. The neutralization of unreacted benzoic acid is conducted by washing with weak alkaline solution. The disadvantage of this method is the use of benzoic acid in the amount lower than stoichiometric relationship which leads to the reduction of the yield of the target product. Moreover, the disadvantage is the duration of conducting the esterification process.

The application US2007027240 discloses a method of production of asymmetrical ester by esterification of aliphatic and aromatic acids by glycol in the presence of catalyst. The esterification is taken place in the presence of xylene as entrainer. In the application are no information about the achieved results of the esterification process: the final product yields, the residual content of benzoic acid, the content of monoester, etc. are not specified. Moreover, there is no information that the problem of benzoic acid sublimation has been solved.

The application US20160264509 discloses a method of production of ester by esterification of acids by alcohol in the presence of organic sulfonic acid catalyst. The esterification can be carried out in the presence of a solvent selected from a wide group of compounds but the examples of using the solvent are absent.

It is known that in order to increase the conversion of the starting products due to the shift of equilibrium, as well as in order to reduce the duration of the esterification process it is necessary to use higher temperatures (higher than 200-220°C). However the excessive increase of the temperature at the beginning of the esterification process leads to intensive carry-over of benzoic acid from the reactor due to sublimation thereof with the fumes of water forming in the esterification process. As a result benzoic acid is crusted in the form of crystals on the equipment, in particular, on outlet tubes and separator, which reduces the process processability since the benzoic acid deposits reduce the effectiveness of heat transmission. Furthermore the equipment for production of benzoic acid esters requires repetitive shut-downs for purification of the equipment from the benzoic acid deposits.

It is also known that for the shift of equilibrium toward the formation of the desired product, one of the reagents should be added in excess. Thus in order to increase the yield of the ester based on benzoic acid and glycol in the esterification process, benzoic acid should be added with a stoichiometric excess. However it should be noted that excessive addition of benzoic acid leads to the higher amount of the unreacted residues thereof in the desired product, which in turn has a negative impact on the product quality, in particular, during further use thereof as a plasticizers. Thus it is important to remove the residues of unreacted benzoic acid from the reaction mass comprising a desired product, namely, ester.

In order to solve said problem in patents CN103739494 and CN103086891 the reaction mass is washed with the solutions of neutralizing agents, namely, alkaline metals hydroxides and carbonates. Said method is the most common and allows removing benzoic acid from the desired product by neutralization thereof with high effectiveness. However the use of neutralizing agents leads to the formation of a large amount of water flow contaminated by organic salts. At that, the higher is the concentration of benzoic acid in the reaction mass, the higher is the flow rate of neutralizing agent and the concentration of organic salt in the water flow. Also the disadvantage of the use of neutralizing agents is the fact that the neutralization of benzoic acid leads to the situation when the removal thereof and the re-use in the esterification process becomes impossible.

The use of vacuum distillation of benzoic acid from the reaction mass is also ineffective in view of the fact that benzoic acid is a sublimating substance, which is capable of crusting on the surface of the equipment used in the esterification process, covering the reactor and pipeline surfaces gradually blocking them which prevents further discharge of the acid.

Thus the development of the method for production of esters based on benzoic acid and alcohol, wherein the reduction of duration of the esterification process, as well as the effective purification of the obtained desired product, and the removal of unreacted benzoic acid is of high priority, important and uncommon technical problem of high priority.

### SUMMARY OF THE INVENTION

The objective of the present invention is to develop a method for preparing benzoic acid esters which provides high selectivity of the process and high yield of the desired product, as well as the reduction of duration of the esterification process. Another objective of the present invention is to develop a method for preparing esters by esterification of benzoic acid and aliphatic alcohol, selected from the group comprising monohydric alcohols with a number of carbon atoms from 6 to 12, or polyhydric alcohol with a number of carbon atoms from 2 to 10, wherein the effective removal of unreacted benzoic acid from the reaction mass and further recycling thereof to the esterification step is provided.

The technical result of the present invention is the reduction of the duration of the esterification process up to 3 hours, the improvement of the process processability, namely, reduction of shut-downs for purification of the equipment, and preventing reduction of heat transmission due to eliminating of deposits of sublimating benzoic acid on the surface of the equipment, and the increase of selectivity of the process for preparing benzoic acid esters up to 99.3%, and the yield of the desired product up to 99.5%.

The technical result is comprised also in the realization of the method for preparing benzoic acid esters without substantial losses of unreacted benzoic acid due to the providing the effective removal thereof from the reaction mass and further recycling to the step of esterification. The additional technical result achieved at the realization of the present invention is the improvement of ecological aspects of the production which relates to the reduction of water flows at the stage of neutralization of the residues of unreacted benzoic acid due to intensification of the process of removal thereof, as a result of which the acid concentration in the reaction mass is reduced by more than 4-6 times. Due to this fact the amount of neutralizing agent, as well as washings forming the water flow are reduced significantly, which in turn reduces the ecological loading.

This technical problem is solved and the achievement of the required technical result is provided due to conducting a process of preparing benzoic acid esters by esterification of said acid and monohydric and polyhydric aliphatic alcohols, selected from the group comprising monohydric alcohols with a number of carbon atoms from 6 to 12, or polyhydric alcohol with a number of carbon atoms from 2 to 10, in the presence of metal containing catalyst and a solvent based on aromatic compound, under the stepwise buildup of the temperature of the process from the temperature of charging the starting components to 180-220°C, further to 200-220°C, and then to 240-250°C. At that after conducting esterification process, removal and further recycling of unreacted benzoic acid is carried out by combined vacuum distillation with aromatic solvent.

The authors of the present invention have surprisingly found that the use of aromatic solvent in the esterification step allows for a short period of time (up to 3 hours) to reach the desired high temperatures (240-250°C), avoiding the distilling of benzoic acid with the vapors of water evolving in the process of esterification. Moreover the use of aromatic solvent allows to effectively remove the residual unreacted benzoic acid from the reaction mass after the esterification, which can be connected with the forming of a mixture comprising mainly associates of benzoic acid and aromatic solvent which have a lower boiling temperature in contrast to individual compounds.

### DESCRIPTION OF THE DRAWINGS

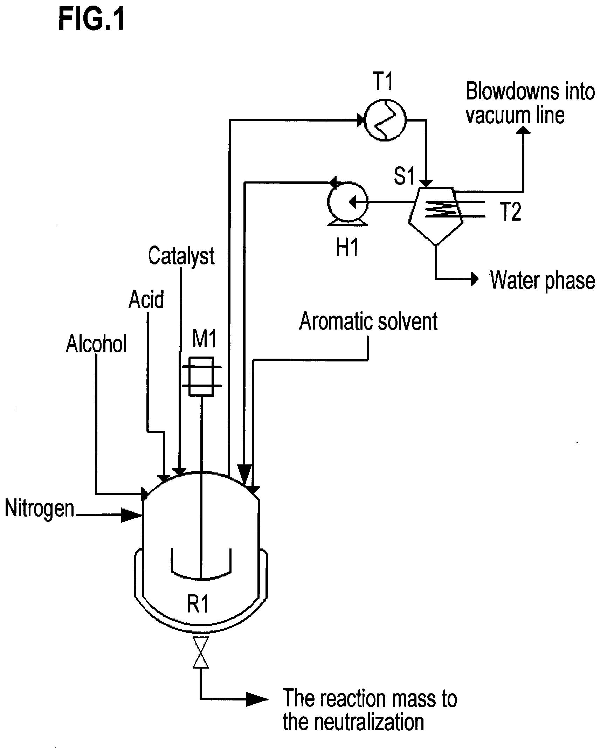

Fig. 1 presents the scheme of the apparatus for producing ester based on benzoic acid and alcohol selected from the group comprising monohydric alcohols with a number of carbon atoms from 6 to 12, or polyhydric alcohol with a number of carbon atoms from 2 to 10, according to the method of the present invention wherein R1- esterification reactor, M1- stirring device, T1- condenser, S1- separator, T2- heat exchanger, H1-circulation pump.

### DETAILED DESCRIPTION OF THE INVENTION

The detailed description of different aspects and options of the implementation of the present invention is further presented.

According to the present invention a method of preparing benzoic acid esters comprises the following steps:
a) esterification of benzoic acid with alcohol, selected from the group comprising monohydric aliphatic alcohols with a number of carbon atoms from 6 to 12, or polyhydric alcohol with a number of carbon atoms from 2 to 10, in the presence of metal containing catalyst and in a media of aromatic solvent at the step-wise increase of the process temperature up to 180-200°C, further up to 200-220°C and then up to 240-250°C to obtain reaction mixture, comprising ester, unreacted benzoic acid and aromatic solvent;
b) vacuum distillation of unreacted benzoic acid together with aromatic solvent from the reaction mixture obtained in step (a) to obtain crude ester;
c) recycling distilled unreacted benzoic acid obtained in step (b) optionally together with aromatic solvent to the esterification step;
d) neutralization of the benzoic acid residues in crude ester and water washing thereof with water;
e) filtration and drying of ester.

### Step (a). Esterification

To prepare esters according to the present invention as starting compounds for esterification reaction the use in made of benzoic acid and alcohol, selected from the group comprising monohydric aliphatic alcohols with a number of carbon atoms from 6 to 12, or polyhydric alcohol with a number of carbon atoms from 2 to 10.

As monohydric alcohols one can use high boiling alcohols with a number of carbon atoms from 6 to 12, in particular, hexanol, octanol, dodecanol, 2-ethylhexanol, etc.

As polyhydric alcohol one can use a diatomic alcohol: glycols having from 2 to 10 carbon atoms, in particular, ethylene glycol, diethylene glycol, triethylene glycol, mono-, di- or tripropylene glycol, butylene glycol, 2-ethylhexane-1,3-diol, 2,2,4-trimethylpentane-1,3-diol, 2-methylpentane-2,4-diol, 3-methylpentane-1,5-diol, 2,2-dimethylpropane-1,3-diol, 1,2-cyclohexane dimethanol, 1,4-cyclohexane dimethanol. Preferably, the use is made of diethylene glycol, dipropylene glycol, and triethylene glycol. As polyhydric alcohol one can use also triatomic alcohols, in particular, glycerol, tetratomic alcohols, in particular, pentaerythritol.

The process of esterification of benzoic acid with alcohol, selected from the group comprising monohydric aliphatic alcohols with a number of carbon atoms from 6 to 12, or polyhydric alcohol with a number of carbon atoms from 2 to 10, is conducted in the presence of metal containing catalyst in the capacity of which the use is made of any esterification metal containing catalyst known from the prior art. The examples of such catalysts include, but are not limited to: titanium compounds, tin compounds, etc. The preferable are titanium (IV) compounds, for example, titanium tetraisopropoxide, titanium tetraisobutoxide and compositions obtained by the reaction between titanium orto-ester and alcohol, acid and/or base. The amount of the catalyst used is from 0.01 to 1% based on the weight of the reaction system, preferably, from 0.04 to 0.06% based on the weight of the reaction system.

Preferably the esterification of benzoic acid with alcohol, selected from the group comprising monohydric aliphatic alcohols with a number of carbon atoms from 6 to 12, or polyhydric alcohol with a number of carbon atoms from 2 to 10, is conducted at the molar ratio benzoic acid:alcohol from 1:1 to 3:1, preferably from 2:1 to 2.5:1, more preferably from 2.05:1 to 2.2:1.

In order to achieve the claimed technical result, the process of esterification of benzoic acid with alcohol, selected from the group comprising monohydric aliphatic alcohols with a number of carbon atoms from 6 to 12, or polyhydric alcohol with a number of carbon atoms from 2 to 10, in the presence of metal containing catalyst should be obligatory carried out in a media of aromatic solvent.

The term aromatic solvent in the present invention means carbocyclic compound the molecule of which has cyclic group having six carbon atoms named benzene or aromatic ring. At that, said carbocyclic compound can be unsubstituted, or can have one or more substituents.

As substituents can be used: alkyl substituents having not more than 7 carbon atoms, such as methyl, ethyl or isopropyl, and/or alkoxy substituants having not more that 7 carbon atoms, such as methoxy- or ethoxy group.

Thus as aromatic solvent according to the present invention the use is made of the compound of the general formula: 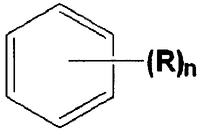 Wherein
R- is alkyl and/or alkoxy substituent having not more than 7 carbon atoms;
n- number of substituents from 0 to 6.

It is preferable to use aromatic solvent with the boiling temperature from 110 to 200°C. The use of such solvent in the esterification process allows to quickly achieve high temperatures (240-250°C) without increasing the pressure. Moreover it is preferably to use aromatic solvent providing full solvability used in the benzoic acid esterification process.

The aromatic solvents comprising two or more benzene rings, as well as carbocyclic compounds, the cycle thereof comprising, along with carbon, heteroatoms, for example, nitrogen, oxygen, sulfur, are not used in the present invention.

According to the present invention as aromatic solvent one can use benzene, toluene, ethyl benzene, cumene, anisole, and any xylene isomer (orto- xylene, para-xylene and metha- xylene), as well as mixtures thereof. The best technical result is achieved when using any xylene isomer or the mixture thereof as aromatic solvent.

The aromatic solvent can be charged into the esterification step in the form of benzoic acid solution. The optimum consumption of aromatic solvent in the process according to the present invention is from 0.01 to 1 l/hour per liter of reaction mass, preferably, from 0.1 to 0.5 l/hour per liter of reaction mass.

According to the present invention preferably aromatic solvent is added in the amount at least 0.05 kg/kg of reaction mass, preferably, at least 0.2 kg/kg of the reaction mass.

The esterification process of benzoic acid with alcohol, selected from the group comprising monohydric alcohols with a number of carbon atoms from 6 to 12, or polyhydric alcohol with a number of carbon atoms from 2 to 10, is conducted at the elevated temperature, preferably at atmospheric pressure in the inert media. The optimum temperature depends on the nature and concentration of the catalyst used. When using the catalysts based on titanium compounds, the esterification reaction is conducted at heating the reaction mixture up to 180-200°C, and further raising the temperature by 10°C each 30 min. till the temperature reaches 240-250°C. Thus the preferable rate of temperature increase of the reaction mixture is from 0.3 to 0.5°C/min. Said temperature mode allows avoiding intensive distillation of benzoic acid with water, and further removal from reaction media at the beginning of the esterification process. At that the use of aromatic solvent provides quick achievement of high temperatures compared with a process without such solvent which allows to reduce the duration of esterification reaction. At that the pressure is from 80 to 150 kPa, preferably, from 90 to 110 kPa, more preferably, 100-102 kPa.

It is preferable to conduct the esterification process in the media of inert gas to prevent side thermal-oxidative processes. As inert gas one can use, for example, argon, nitrogen, or helium.

The esterification process is conducted in the reaction vessel which is a reactor equipped by the heating element with a stirring device in the form of a mixer or a circulation pump, and vapor delivery tube which is used for removal of reaction water from the reactor in the form of azeotropic mixture with alcohol.

The benzoic acid, alcohol, esterification metal containing catalyst and aromatic solvent are charged into the reactor in any sequence both together and separately. Preferably the components are charged in the following sequence: 1) benzoic acid, 2) alcohol, 3)optionally adsorbent, 4) aromatic solvent, 5) metal containing catalyst. Benzoic acid can be charged in the form of powder, pellets, crystallisat or melt. It is also possible to charge benzoic acid in aromatic solvent. In order to reduce the time of the process it is preferable to begin the heating during the charge of the starting components. The catalyst can be charged into the reactor in the pure form, in the form of suspension or in the form of solution, at any step of esterification. The catalyst used in the esterification process is added in the desired amount simultaneously or batchwise in the form of two or more parts.

In order to improve the colority of the desired product in the step of esterification one can use adsorbents capable of adsorption of coloring impurities. In particular, activated carbon, celite, perlite, etc. can be used as adsorbents. The amount of adsorbent is from 0.01 to 2% by weight based on the weight of the reaction mass, preferably, from 0.1 to 1% by weight based on the weight of the reaction mass. The adsorbent is charged into the esterification reactor separately and simultaneously with other starting components.

The reaction mixture obtained by the esterification process described above comprises a desired product: ester, unreacted benzoic acid and aromatic solvent.

### Step (b). Vacuum distillation of unreacted benzoic acid together with aromatic solvent.

After the esterification reaction is completed (the end of the reaction is defined by the amount of separated water) the step of recovering of excess of acid from the ester is conducted, the amount of which in the reaction mass is from 8 to 9% by weight. For this purpose the method of vacuum distillation of benzoic acid from the reaction mass is used at the temperature from 240 to 250°C under the pressure lower than atmospheric. Usually such method comes with a number of problems, one of which is that benzoic acid sublimates and deposits on the cold walls of the equipment used in the method for preparing ester, in particular on the cold walls of the steam pipeline, which prevents further recovery of said acid.

The authors of the invention surprisingly found that vacuum distillation of unreacted benzoic acid in the presence of aromatic solvent, preferably, xylene prevents the deposition of benzoic acid on the surface of equipment, as well as allows a more effective realization of the recovery thereof. Thus the use of aromatic solvent, in particular, xylene in the process of vacuum distillation of benzoic acid allows to effectively realize the recovery thereof by reducing the amount thereof in the reaction mass by more than 85%.

From the prior art known are examples of the use of aromatic solvents in the esterification process including xylene. Known also is the esterification method from the patent EP 1664178 wherein aromatic solvent (xylene) is used as entrainer in order to remove the water formed, and the removal of unreacted acid from reaction mass is conducted by neutralization with sodium hydroxide solution. However from the prior art it is not known to use aromatic solvent including xylene in order to more effectively conduct the esterification process of the benzoic acid with alcohol and subsequent removal and recycling of the unreacted benzoic acid residues.

According to the present invention the aromatic solvent is charged into the reactor at the step of esterification separately or together with other starting components. When entering the reactor the aromatic solvent evaporates and is removed from the reaction zone through vapor pipeline. Due to the fact that benzoic acid is dissolved in the aromatic solvent at the temperature of the esterification reaction from 200 to 250°C, the benzoic acid in the form of vapor-liquid mixture is freely removed together with aromatic solvent. Further the separated mixture of benzoic acid and aromatic solvent is devaporated and cooled to room temperature from 20 to 25°C whereby the deposition of benzoic acid in the form of crystals occurs.

The step of benzoic acid distillation is conducted at the temperature from 240 to 250°C and pressure from 0.1 to 30 kPa, preferably from 2 to 15 kPa.

### Step (c) Recycling unreacted benzoic acid

According to the present invention it is advantageous to conduct a process in such a way that the separated benzoic acid solution optionally with aromatic solvent is used repeatedly by the recycling thereof back to the esterification reactor. For this purpose the mixture of aromatic solvent and benzoic acid distilled in step (b) is heated in order to dissolve the acid crystals. It is heated to the temperature at which benzoic acid goes over to the solution, preferably, to the temperature from 20 to 150°C, more preferably, from 40 to 80°C. Further the obtained solution of benzoic acid in the aromatic solvent is recycled for the repeated use in the esterification reactor.

According to the present invention the use of xylene in the process of vacuum distillation of unreacted benzoic acid allows to significantly reduce the amount thereof in the reaction mixture by more than 85% (by weight) based on the total amount of unreacted acid. The rest amount of benzoic acid in the reaction mass is removed by neutralization.

### Step (d). Neutralization of the benzoic acid residues in crude ester.

The purification of the reaction mass comprising crude ester after conducting distillation of the unreacted benzoic acid includes also neutralization of the residual of benzoic acid in crude ester by neutralization agent which is an aqueous solution of alkali agent. As an alkali agent the use is made of any base known from the prior art, for example, sodium hydroxide, sodium carbonate, sodium silicate, potassium hydroxide, calcium carbonate or magnesium carbonate, etc. The amount of neutralization agent is usually amounts from 0.5 to 3 mol per mol of the residual acid, preferably, from 1 to 1.5 mol per mol of the residual acid. It is preferable to conduct a neutralization process at the temperature from 50 to 90°C and atmospheric pressure.

### Step (e). Filtration and drying of ester.

The obtained product which is a crude ester is filtered in order to separate the adsorbent in case of using it and the residues of the catalyst hydrolyzed in the step of neutralization. The filtration process is carried out in any apparatus known from the prior art -filters- equipped by porous filter barriers capable to separate solid phase from the desired product. The filtration of the crude ester is conducted at the temperature from 50 to 90°C.

In order to remove from the ester obtained based on benzoic acid and glycol such components as water, solvent or the vapors thereof the process of drying said ester is conducted. The drying process can be carried out by physical methods usually used for separating and purification of organic compounds (extraction, salting-out, fractional and azeotropic distillation, volatilization, etc.), as well as by using drying reagents which eliminate moisture due to adsorption, formation of hydrates or chemical reaction with water.

Preferably the drying is conducted at the temperature from 105 to 115°C and the pressure from 5 to 10 kPa.

The product obtained according to the present invention is an ester based on benzoic acid and alcohol, selected from the group comprising monohydric aliphatic alcohols with a number of carbon atoms from 6 to 12, or polyhydric alcohol with a number of carbon atoms from 2 to 10, and is characterized by the purity not less than 95%. This product can be used as plasticizer for polyvinylchloride (PVC), as well as in other fields including paints, inks or lacquers, plastisols, adhesives or components of adhesives, sealing compositions.

The invention is clarified in more detail in Fig.1 wherein the technological scheme of preparing ester based on benzoic acid and alcohol, selected from the group comprising monohydric alcohols having a number of carbon atoms from 6 to 12, or polyhydric alcohol having a number of carbon atoms from 2 to 10, is presented. The scheme presented in Fig.1 is an example of implementation of the present invention, and does not limit it by specified details of the present disclosure.

The ester based on benzoic acid and alcohol, selected from the group comprising monohydric alcohols having a number of carbon atoms from 6 to 12, or polyhydric alcohol having a number of carbon atoms from 2 to 10 is obtained by sequence of the steps furnished below.
1) According to the method of the present invention the benzoic acid and alcohol, selected from the group comprising monohydric alcohols having a number of carbon atoms from 6 to 12, or polyhydric alcohol having a number of carbon atoms from 2 to 10, catalyst - titanium tetraisopropoxide and optionally adsorbent are charged into the esterification reactor R-1, equipped with heating element and a stirrer (M1). The aromatic solvent, preferably, xylene, is also charged into the esterification reactor R-1. At that the aromatic solvent can be added into the esterification reactor R-1 in a pure form, or in the form of the solution of benzoic acid. Said solvent is added into the esterification reactor R-1 together with other components before the heating step, or separately after the desired initial temperature of (180-200°C) in the reactor R-1 is reached.
2) Further the reaction mixture of the starting components in reactor R-1 is heated to the initial temperature 180-200°C and the esterification process is conducted at the atmospheric pressure in the media of inert gas, preferably nitrogen.
3) When the temperature of 180-200°C is achieved, the water vapors and aromatic solvent with benzoic acid enter through vapor pipeline the condenser T1, wherein they are evaporated and enter S1- separator.
4) The mixture is separated into two phases in separator S1: aqueous bottom phase and upper organic phase.
5) The bottom aqueous phase is removed batchwise (pour out) from S1-separator.
6) The upper organic phase which is a solution of benzoic acid in aromatic solvent is directed back to esterification reactor R1 from separator S1 by circulating pump H1. The circulation of the aromatic solvent is avoided the benzoic acid crystals in the equipment and acid is returned back to the esterification reactor
7) In order to accelerate the process and to improve selectivity of production of the desired product in an hour after the beginning of the heating, the temperature in the esterification reactor R1 is increased from 180-200°C to 200-220°C, in 2 hours after the beginning of the heating the temperature is increased up to 240-250°C.
8) In 3 hours the esterification reaction is completed, and the distillation of unreacted benzoic acid is conducted in the esterification reactor. In this regard, under constant recycling of aromatic solvent with the optimum flow through the pump H1, the pressure in separator S1 is reduced to 15 kPa by vacuum pump, in 30 min more the pressure is reduced to 5 kPa. During the pressure reduction the aromatic solvent begins to evaporate intensively from the reactor R1 entering first through vapor pipeline the condenser T1, then the separator S1. Due to good solubility of benzoic acid in aromatic solvent at 250°C, the unreacted benzoic acid is also carried away through the vapor pipeline into condenser in the form of vapor-liquid mixture together with aromatic solvent.
9) In separator S1 the solution of benzoic acid in aromatic solvent is cooled in the heat exchanger T2. Because of limited solubility of benzoic acid precipitates from the aromatic solvent in the form of solid phase. The benzoic acid -deficient aromatic solvent is directed back to the reactor R1 by circulating pump H1. The cycle evaporation- condensation is repeated.
   At that according to the present invention in order to prevent nonrecoverable losses of unreacted benzoic acid the recycling thereof to the esterification step is conducted. In this regard, the mixture comprised in separator S1 is heated to the temperature from 60 to 80°C for dissolving the benzoic acid in aromatic solvent. Further the solution of benzoic acid in aromatic solvent from the separator S1 is pumped to reactor R1 by the pump H1 along the thermal insulated pipeline for repeated use in the esterification process.
   It should be noted that the benzoic acid separated according to the present invention comprised in separator S1 can be discharged in the form of saturated solution and used in another process.
10) In 4 hours after the beginning of the heating the reaction mass is discharged from the reactor R1. The obtained ester is treated by the aqueous solution of neutralizing agent for removing the residues of benzoic acid. The conditions and ratios of reagents can also be chosen from the prior art.
11) Further the ester is filtered in order to separate the solid phase, in particular, adsorbent, in case of use thereof, and is dried in order to remove the water and solvent residues to obtain ester based on benzoic acid which is the end product.

The present invention will be described in detail with reference to the examples furnished below. These examples are set forth only for the illustration of the present invention, and do not limit it.

### Examples of the invention implementation

### Example 1.

In steel esterification reactor with the volume of 15 1 equipped with a heating element and a stirring device, 3550 g of benzoic acid, 1500 g of diethylene glycol are charged, 13 g of titanium (IV) isopropoxide are added.

Before the heating treatment, xylene in the amount of 2500 g is added to separator. The reaction is conducted at the constant feed of xylene into the reaction mass by the circulating pump from the separator with the rate of 3 l/hour. The reaction mass is heated to the temperature 200°C, in an hour after the beginning of the heating the temperature is raised to 220°C, in two hours after the beginning of the heating the temperature is raised to 240°C.

In 3 hours after the beginning of the heating the pressure in reactor is reduced to 15 kPa, in 30 min more the pressure in reactor is reduced to 5 kPa. In 4 hours after the beginning of the heating the reaction mass is cooled.

From the esterification reactor 4400 g of reaction mass comprising 0.5% by weight of benzoic acid, 0.3 % by weight of diethylene glycol benzoate and 99.0% by weight of diethylene glycol dibenzoate are discharged. From the separator an aqueous phase comprising 785 g of water, 8 g of benzoic acid, as well as organic phase comprising 1720 g of xylene, 365 g of benzoic acid, is discharged.

### Example 2 (comparative).

In steel esterification reactor with the volume of 15 1 equipped with a heating element and a stirring device, 6500 g of benzoic acid, 2650 g of diethylene glycol are charged, 23 g of titanium (IV) isopropoxide are added.

Before the heating treatment, xylene in the amount of 2500 g is added to separator. The reaction mass is heated to the temperature 200°C. The esterification process is conducted at atmospheric pressure. In 4 hours after the beginning of the heating the temperature is raised to 220°C, in 6 hours after the beginning of the heating the temperature is raised to 240°C, in 8 hours after the beginning of the heating the reaction mass is cooled.

From the esterification reactor 8290 g of reaction mass comprising 7.8% by weight of benzoic acid, 9 % by weight of diethylene glycol benzoate and 83.2% by weight of diethylene glycol dibenzoate are discharged. From the separator a phase comprising 856 g of water, 15 g of benzoic acid, is discharged.

### Example 3(comparative).

The example is conducted according to example 2 except that 4.5 g of titanium (IV) isopropoxide are added. The reaction mass is heated to the temperature 200°C, in an hour after the beginning of the heating the temperature is raised to 220°C, in two hours after the beginning of the heating the temperature is raised to 240°C, in 4 hours after the beginning of the heating the reaction mass is cooled.

From the esterification reactor 8255 g of reaction mass comprising 7.6% by weight of benzoic acid, 4.5 % by weight of diethylene glycol benzoate and 87.5% by weight of diethylene glycol dibenzoate are discharged. From the separator a phase comprising 878 g of water, 35 g of benzoic acid, is discharged.

### Example 4.

Example is conducted according to example 1 except that in 3 hours after the beginning of the heating the pressure in reactor is not reduced.

From the esterification reactor 8590 g of reaction mass comprising 4.6% by weight of benzoic acid, 4% by weight of xylene, 1.7% by weight of diethylene glycol benzoate and 89.7% by weight of diethylene glycol dibenzoate are discharged. From the separator a phase comprising 890 g of water, 8 g of benzoic acid, as well as organic phase comprising 2151 g of xylene, 35 g of benzoic acid, is discharged.

### Example 5.

The example is conducted according to example 1 except that in 3 hours after the beginning of the heating the pressure in reactor is reduced to 50 kPa.

From the esterification reactor 8270 g of reaction mass comprising 4% by weight of benzoic acid, 1.3 % by weight of xylene, 1.5% by weight of diethylene glycol benzoate and 93.3% by weight of diethylene glycol dibenzoate are discharged. From the separator an aqueous phase comprising 892 g of water, 8 g of benzoic acid, as well as organic phase comprising 2170 g of xylene, 100 g of benzoic acid, is discharged.

### Example 6.

The example is conducted according to example 1 except that in 3 hours after the beginning of the heating the pressure in reactor is reduced to 10 kPa.

From the esterification reactor 8020 g of reaction mass comprising 2.5% by weight of benzoic acid, 0.2 % by weight of xylene, 1% by weight of diethylene glycol benzoate and 96% by weight of diethylene glycol dibenzoate are discharged. From the separator an aqueous phase comprising 895 g of water, 8 g of benzoic acid, as well as organic phase comprising 2256 g of xylene, 210 g of benzoic acid, 7 g of diethylene glycol benzoate and 44 g of diester, is discharged.

### Example 7.

The example is conducted according to example 1 except that the reaction is conducted at the constant feed of xylene into the reaction mass by the circulating pump from the separator with the rate of 6 l/hour.

From the esterification reactor 7620 g of reaction mass comprising 3.4% by weight of benzoic acid, 0.8 % by weight of diethylene glycol benzoate and 95.6% by weight of diethylene glycol dibenzoate are discharged. From the separator an aqueous phase comprising 895 g of water, 9 g of benzoic acid, as well as organic phase comprising 2260 g of xylene, 150 g of benzoic acid, and 500 g of diethylene glycol dibenzoate, is discharged.

### Example 8.

The example is conducted according to example 1 except that the reaction is conducted at the constant feed of xylene into the reaction mass by the circulating pump from the separator with the rate of 1 l/hour.

From the esterification reactor 8230 g of reaction mass comprising 4.3% by weight of benzoic acid, 0.6 % by weight of diethylene glycol benzoate and 93.5% by weight of diethylene glycol dibenzoate are discharged. From the separator an aqueous phase comprising 895 g of water, 10 g of benzoic acid, as well as organic phase comprising 2220 g of xylene, 50 g of benzoic acid, is discharged.

### Example 9.

The example is conducted according to example 1 except that as a solvent ethyl benzene is used.

From the esterification reactor 8070 g of reaction mass comprising 3% by weight of benzoic acid, 0.5 % by weight of diethylene glycol benzoate and 96.5% by weight of diethylene glycol dibenzoate are discharged. From the separator an aqueous phase comprising 895 g of water, 7 g of benzoic acid, as well as organic phase comprising 2320 g of ethylbenzene, 160 g of benzoic acid, is discharged.

### Example 10. The general neutralization example.

In steel reactor with the volume of 15 l equipped with a heating element and a stirring device, 7900 g of reaction mass obtained in example 1 are charged. 400 g of 5% by weight aqueous solution of sodium hydroxide are added. The reaction mass is heated to the temperature 80°C, and the process is conducted under stirring and atmospheric pressure for 1 hour. After 1 hour the product is dried and filtered. 7750 g of the product comprising 0.6% by weight of diethylene glycol benzoate and 99.4 by weight of diethylene glycol dibenzoate is obtained.

### Example 11.

In steel reactor with the volume of 15 l equipped with a heating element and a stirring device, 6000 g of benzoic acid, 2700 g of diethylene glycol are charged, 4.5 g of titanium (IV) isopropoxide are added. In separator a mixture of 500 g of benzoic acid and 2500 g of xylene is charged. The reaction mass is heated to the temperature 200°C, the process is conducted at the constant feed of xylene into the reaction mass by the circulating pump from the separator with the rate of 3 l/hour. The sample of water from separator S1 is periodically collected for gravimetric analysis at atmospheric pressure.

In an hour after the beginning of the heating the temperature is raised to 220°C, in two hours after the beginning of the heating the temperature is raised to 240°C, in 3 hours after the beginning of the heating the pressure in reactor is reduced to 15 kPa, in 30 min more the pressure in reactor is reduced to 5 kPa. In 4 hours after the beginning of the heating the reaction mass is cooled, and the product is discharged from ehterificator.

From the esterification reactor 7970 g of reaction mass comprising 1.6% by weight of benzoic acid, 0.4 % by weight of diethylene glycol benzoate and 97.7% by weight of diethylene glycol dibenzoate are discharged. From the separator a phase comprising 895 g of water, 6 g of benzoic acid, as well as organic phase comprising 2500 g of xylene, 280 g of benzoic acid, is discharged.

The basic data for the methods according to examples 1-11 are presented in table 1.

**Table 1. The results of esterification of benzoic acid with alcohol according to the examples 1-11**

| Parameter | Example 1 | | Example 2 | | Example 3 | | Example 4 | | Example 5 | | Example 6 | | Example 7 | | Example 8 | | Example 9 | | Example 11 | |
|---|---|---|---|---|---|---|---|---|---|---|---|---|---|---|---|---|---|---|---|---|
| Step Temperature, °C | 1. | 200 | 1. | 200 | 1. | 200 | 1. | 200 | 1. | 200 | 1. | 200 | 1. | 200 | 1. | 200 | 1. | 200 | 1. | 200 |
| | 2. | 220 | 2. | 220 | 2. | 220 | 2. | 220 | 2. | 220 | 2. | 220 | 2. | 220 | 2. | 220 | 2. | 220 | 2. | 220 |
| | 3. | 240 | 3. | 240 | 3. | 240 | 3. | 240 | 3. | 240 | 3. | 240 | 3. | 240 | 3. | 240 | 3. | 240 | 3. | 240 |
| Step Pressure, kPa | 1. | 100 | 100 | | 100 | | 100 | | 1. | 100 | 1. | 100 | 1. | 100 | 1. | 100 | 1. | 100 | 1. | 100 |
| | 2. | 15 | | | | | | | 2. | 5 | 2. | 10 | 2. | 15 | 2. | 15 | 2. | 15 | 2. | 15 |
| | 3. | 5 | | | | | | | | | | | 3. | 5 | 3. | 5 | 3. | 5 | 3. | 5 |
| Total reaction time, h | 4 | | 8 | | 4 | | 4 | | 4 | | 4 | | 4 | | 4 | | 4 | | 4 | |
| Solvent | Xylene | | - | | - | | Xylene | | Xylene | | Xylene | | Xylene | | Xylene | | ethylbenzene | | Xylene | |
| Xylene consumption, l/h | 3 | | - | | - | | 3 | | 3 | | 3 | | 6 | | 1 | | 3 | | 3 | |
| Pressure at the distillation stage, kPa | Up to 5 | | 100 | | 100 | | 100 | | Up to 50 | | Up to 10 | | Up to 5 | | Up to 5 | | Up to 5 | | Up to 5 | |
| Yield of the product (ester), % | 99.5 | | 88 | | 92 | | 98.8 | | 98.9 | | 98.7 | | 93 | | 99.2 | | 99.2 | | 99.5 | |
| Yield of the monoester by-product, % | 0.5 | | 6.8 | | 3.3 | | 1.3 | | 1 | | 0.6 | | 0.4 | | 0.2 | | 0.2 | | 0.5 | |
| Unreacted benzoic acid residue, % | 0.5 | | 7.8 | | 7.6 | | 4.6 | | 4 | | 2.5 | | 3.4 | | 4.3 | | 3 | | 1.6 | |

Example 1 illustrates the achievement of the technical result described in the present invention at the optimal conditions of conducting the process. The use of xylene both in the step of esterification, and in the step of removing of unreacted benzoic acid from the reaction mass allows to increase the yield of the product up to 99.5%. The selectivity of the esterification process at that is 99%.

In example 2 the method of preparing esters was conducted according to the standard procedure without the use of aromatic solvent and under the slower heating of the reaction mixture.

The method disclosed in example 2 is characterized by the duration of the process (8 hours), a higher amount of unreacted benzoic acid in the reaction mass, and a by-produced monoester.

The realization of the esterification at higher temperatures without using aromatic solvent as in example 3, leads to severe contamination of the equipment by the crystals of benzoic acid which makes such process technologically unfeasible.

The use of higher pressure (10-100 kPa) upon separation of diester and unreacted benzoic acid as shown in examples 4, 5 and 6, leads to ineffective removal of benzoic acid from the reaction mass.

It should be noted that the use of active circulation of xylene leads to the dramatic drop in the yield of the desired product because of high dilution of reaction mass, and therefore, to carrying-over of the benzoic acid from reactor R1 into separator S1 (Example 7). At that the use of a weaker xylene circulation in comparison with Example 1 does not reduce the yield of diester significantly, but deteriorates the process of effective removal of unreacted benzoic acid.

Example 9 is furnished as a comparison example wherein as a diluent the use was made of ethylbenzene. Ethylbenzene allows to conduct the removal of unreacted benzoic acid from the reaction mass, but the effectiveness thereof is lower than in case of using xylene.

Example 11 illustrates the possibility of the repeated use of benzoic acid distilled from the reactor by heating thereof in separator and recycling into the reactor to the esterification step.

Thus from examples of implementation of the invention it is obvious that the best technical result is achieved upon production of esters based on benzoic acid according to the method furnished in the present invention.

## Claims

1. A method for preparing benzoic acid esters comprising the following steps:
a) esterification of benzoic acid and alcohol, selected from the group comprising monohydric aliphatic alcohols having a number of carbon atoms from 6 to 12, or polyhydric alcohol having a number of carbon atoms from 2 to 10, in the presence of metal containing catalyst in a media of an aromatic solvent, under the stepwise buildup of the temperature of the process from the temperature of charging the starting components to 180-200°C, further to 200-220°C, and then to 240-250°C, to obtain the reaction mixture comprising ester, unreacted benzoic acid and aromatic solvent;
b) vacuum distillation of unreacted benzoic acid together with aromatic solvent from the reaction mixture obtained in step (a) to produce crude ester;
c) recycling the unreacted benzoic acid distilled in step (b) optionally together with aromatic solvent to the esterification step;
d) neutralization of the benzoic acid residues in crude ester and washing thereof with water;
e) filtration and drying of ester.

2. The method according to claim 1, wherein monohydric aliphatic alcohol having a number of carbon atoms from 6 to 12, is hexanol, octanol, dodecanol, 2-ethylhexanol.

3. The method according to claim 1, wherein polyhydric alcohol having from 2 to 10 carbon atoms, is a diatomic alcohol or triatomic alcohol, preferably a diatomic alcohol is glycol having from 2 to 10 carbon atoms, in particular, ethylene glycol, diethylene glycol, triethylene glycol, mono-, di- or tripropylene glycol, butylene glycol, 2,2,4-tri-methylpentane-1,3-diol, 1,2-cyclohexandimethanol, 1,4-cyclohexandimethanol, a triatomic alcohol is glycerol, more preferably a diatomic alcohol is glycol having from 2 to 10 carbon atoms, in particular, diethylene glycol, dipropylene glycol, and triethylene glycol.

4. The method according to claim 1, wherein a metal containing catalyst is organic compounds of titanium, tin, zirconium, preferably a metal containing catalyst is the compositions obtained by the reaction between titanium orto-ester and alcohol, acid and/or base or a metal containing catalyst is the titanium tetraisopropoxide or titanium tetraisobutoxide.

5. The method according to claim 1, wherein the amount of a metal containing catalyst is from 0.01 to 1% based on the reaction mass, preferably from 0.04 to 0.06% based on the reaction mass.

6. The method according to claim 1, wherein the esterification of benzoic acid with alcohol, selected from the group comprising monohydric alcohols having a number of carbon atoms from 6 to 12, or polyhydric alcohol having a number of carbon atoms from 2 to 10, is conducted at the molar ratio benzoic acid:alcohol from 1:1 to 3:1, preferably from 2:1 to 2.5:1, more preferably from 2.05:1 to 2.2:1.

7. The method according to claim 1, wherein an aromatic solvent is a carbocyclic compound, the molecule of which has cyclic group having six carbon atoms named benzene or aromatic ring.

8. The method according to claim 7, wherein said carbocyclic compound can be unsubstituted, or can have one or more substituents, the substituents being alkyl substituents having not more than 7 carbon atoms, such as methyl, ethyl or isopropyl, and/or alkoxy substituents having not more that 7 carbon atoms, such as methoxy- or ethoxy group, preferably an aromatic solvent is cumene, toluene, ethyl benzene, chlorbenzene, any xylene isomer (orto-xylene, para-xylene and metha-xylene), as well as the mixtures thereof, more preferably an aromatic solvent is any xylene isomer, or the mixtures thereof.

9. The method according to claim 1, wherein an aromatic solvent has a boiling temperature from 110 to 200°C.

10. The method according to claim 1, wherein an aromatic solvent is charged into the esterification step in the form of solution with benzoic acid.

11. The method according to claim 1, wherein the consumption of aromatic solvent is from 0.01 to 1 1/hour per liter of reaction mass, preferably, from 0.1 to 0.5 1/hour per liter of reaction mass.

12. The method according to claim 1, wherein an aromatic solvent is added in the amount at least 0.05 kg/kg of reaction mass, preferably, at least 0.2 kg/kg of the reaction mass.

13. The method according to claim 1, wherein the rate of temperature increase in the esterification process is from 0.3 to 0.5°C/min.

14. The method according to claim 1, wherein the esterification is conducted at the pressure from 80 to 150 kPa, preferably, from 90 to 110 kPa, more preferably, 100-102 kPa.

15. The method according to claim 1, wherein the esterification is conducted in the media of inert gas.

16. The method according to claim 1, wherein the esterification is conducted at the intensive stirring.

17. The method according to claim 1, wherein an adsorbent is used in the esterification step.

18. The method according to claim 17, wherein an adsorbent is activated carbon, celit, perlite.

19. The method according to claim 17, wherein the adsorbent is used in the amount of from 0.01 to 2% by weight based on the weight of the reaction mass, preferably, from 0.1 to 1% by weight based on the weight of the reaction mass.

20. The method according to claim 1, wherein the vacuum distillation of unreacted benzoic acid together with aromatic solvent is conducted at the temperature from 240 to 250°C.

21. The method according to claim 1, wherein the vacuum distillation of unreacted benzoic acid together with aromatic solvent is conducted at the pressure from 0.1 to 30 kPa, preferably from 2 to 15 kPa.

22. The method according to claim 1, wherein in order to recycle benzoic acid the preliminary heating of the unreacted benzoic acid distilled in step (b), optionally together with an aromatic solvent is conducted, in order to dissolve the acid crystals.

23. The method according to claim 22, wherein the heating is effected to the temperature at which the benzoic acid goes over to the solution, preferably, to the temperature from 20 to 150°C, more preferably, from 40 to 80°C.

24. The method according to claim 1, wherein neutralization of the residual of benzoic acid in crude ester is conducted with an aqueous solution of alkali agent, which is sodium hydroxide, sodium carbonate, sodium silicate, potassium hydroxide, calcium carbonate or magnesium carbonate.

25. The method according to claim 24, wherein the amount of alkali agent is from 0.5 to 3 mol per mol of the residual acid, preferably, from 1 to 1.5 mol per mol of the residual acid.

26. The method according to claim 1, wherein the neutralization process is conducted at the temperature from 50 to 90°C.

27. The method according to claim 1, wherein the neutralization process is conducted at the atmospheric pressure.

28. The method according to claim 1, wherein the filtration of the ester is conducted at the temperature from 50 to 90°C.

29. The method according to claim 1, wherein the drying of ester is conducted at the temperature from 105 to 115°C.

## Patentansprüche

1. Verfahren zur Herstellung von Benzoesäureestern, umfassend die folgende Schritte:
a) Veresterung von Benzoesäure und Alkohol, ausgewählt aus der Gruppe, die einwertige aliphatische Alkohole mit einer Anzahl von 6 bis 12 Kohlenstoffatomen oder mehrwertigen Alkoholen mit einer Anzahl von von Kohlenstoffatomen von 2 bis 10, in Gegenwart eines metallhaltigen Katalysators in einem Medium aus einem aromatischen Lösungsmittel, unter schrittweiser Erhöhung der Temperatur des Verfahrens von der Temperatur der Beschickung der Ausgangskomponenten auf 180-200°C, weiter auf 200-220°C und dann auf 240-250°C, um das Reaktionsgemisch zu erhalten, bestehend aus Ester, nicht umgesetzter Benzoesäure und aromatischem Lösungsmittel;
b) Vakuumdestillation der nicht umgesetzten Benzoesäure zusammen mit aromatischem Lösungsmittel aus dem in Schritt (a) erhaltenen Reaktionsgemisch (a) erhaltenen Reaktionsgemischs zur Herstellung von Rohester;
c) Rückführung der in Schritt (b) destillierten, nicht umgesetzten Benzoesäure gegebenenfalls zusammen mit aromatischem Lösungsmittel in dem Veresterungsschritt;
d) Neutralisation der Benzoesäurereste im Rohester und Waschen desselben mit Wasser;
e) Filtration und Trocknung des Esters.

2. Verfahren nach Anspruch 1,
wobei ein einwertiger aliphatischer Alkohol mit einer Anzahl von Kohlenstoffatomen von 6 bis 12, ist Hexanol, Octanol, Dodecanol, 2-Ethylhexanol.

3. Verfahren nach Anspruch 1,
wobei ein mehrwertiger Alkohol mit 2 bis 10 Kohlenstoffatomen, ein zweiatomiger Alkohol oder dreiatomiger Alkohol ist, vorzugsweise ein zweiatomiger Alkohol ist Glykol mit von 2 bis 10 Kohlenstoffatomen, insbesondere Ethylenglykol, Diethylenglykol, Triethylenglykol, Mono-, Di- oder Tripropylenglykol Glykol, Butylenglykol, 2,2,4-Tri-Methylpentan-1,3-diol, 1,2 Cyclohexandimethanol, 1,4-Cyclohexandimethanol, ein triatomischer Alkohol Glycerin ist, vorzugsweise ein zweiatomiger Alkohol Glykol ist mit 2 bis 10 C-Atomen, insbesondere Diethylenglykol, Dipropylenglykol und Triethylenglykol.

4. Verfahren nach Anspruch 1,
wobei ein metallhaltiger Katalysator organische Verbindungen von Titan, Zinn, Zirkonium ist, vorzugsweise ist ein metallhaltiger Katalysator die Zusammensetzungen, die durch die Reaktion zwischen Titanortoester und Alkohol erhalten werden, Säure und/oder Base erhalten werden, oder ein metallhaltiger Katalysator ist das Titan Tetraisopropoxid oder Titantetraisobutoxid.

5. Verfahren nach Anspruch 1,
wobei die Menge eines metallhaltigen Katalysators von 0,01 bis 1 %, bezogen auf die Reaktionsmasse, vorzugsweise von 0,04 bis 0,06 %, bezogen auf die Reaktionsmasse, beträgt.

6. Verfahren nach Anspruch 1,
wobei die Veresterung von Benzoesäure mit Alkohol, ausgewählt aus der Gruppe, die einwertige Alkohole umfasst mit einer Anzahl von Kohlenstoffatomen von 6 bis 12, oder mehrwertigem Alkohol mit einer Anzahl von Kohlenstoffatomen von 2 bis 10, bei einem molaren Verhältnis von Benzoesäure:Alkohol von 1:1 bis 3:1, vorzugsweise von 2:1 bis 2,5:1, besonders bevorzugt von 2,05:1 bis 2,2:1.

7. Verfahren nach Anspruch 1,
wobei ein aromatisches Lösungsmittel eine carbocyclische Verbindung ist, deren Molekül eine cyclische Gruppe mit sechs Kohlenstoffatomen aufweist, die Benzol oder aromatischer Ring genannt wird.

8. Verfahren nach Anspruch 7,
wobei die carbocyclische Verbindung unsubstituiert sein kann, oder einen oder mehrere Substituenten aufweisen kann, wobei die Substituenten Alkylsubstituenten mit nicht mehr als 7 Kohlenstoffatomen, wie Methyl, Ethyl oder Isopropyl, und/oder Alkoxysubstituenten mit nicht mehr als 7 Kohlenstoffatomen, wie die Methoxy- oder Ethoxygruppe, vorzugsweise ein aromatisches Lösungsmittel Cumol, Toluol, Ethylbenzol, Chlorbenzol, jedes Xylolisomer (orto-xylol, para-xylol und metha-xylol), sowie die Mischungen davon, vorzugsweise ist ein aromatisches Lösungsmittel ein beliebiges Xylolisomer isomer oder deren Gemische.

9. Verfahren nach Anspruch 1,
wobei ein aromatisches Lösungsmittel eine Siedetemperatur von 110 bis 200°C hat.

10. Verfahren nach Anspruch 1,
bei dem ein aromatisches Lösungsmittel in Form einer Lösung mit Benzoesäure in den Veresterungsschritt eingebracht wird.

11. Verfahren nach Anspruch 1,
wobei der Verbrauch von aromatischem Lösungsmittel von 0,01 bis 1 l/Stunde pro Liter der Reaktionsmasse, vorzugsweise von 0,1 bis 0,5 l/Stunde pro Liter Reaktionsmasse.

12. Verfahren nach Anspruch 1,
wobei ein aromatisches Lösungsmittel in einer Menge von mindestens 0,05 kg/kg der Reaktionsmasse, vorzugsweise mindestens 0,2 kg/kg der Reaktionsmasse, zugesetzt wird.

13. Verfahren nach Anspruch 1,
wobei die Geschwindigkeit der Temperaturerhöhungsrate im Veresterungsprozess von 0,3 bis 0. 5°C/min ist.

14. Verfahren nach Anspruch 1,
wobei die Veresterung bei einem Druck von 80 bis 150 kPa, vorzugsweise von 90 bis 110 kPa, noch bevorzugter von 100-102 kPa, durchgeführt wird.

15. Verfahren nach Anspruch 1,
wobei die Veresterung im Medium eines Inertgases durchgeführt wird.

16. Verfahren nach Anspruch 1,
bei dem die Veresterung unter intensivem Rühren durchgeführt wird.

17. Verfahren nach Anspruch 1,
bei dem ein Adsorptionsmittel in der Veresterungsstufe verwendet wird.

18. Verfahren nach Anspruch 17,
wobei ein Adsorptionsmittel Aktivkohle, Celit, Perlit ist.

19. Verfahren nach Anspruch 17,
wobei das Adsorptionsmittel in einer Menge von 0,01 bis 2 Gew.-%, bezogen auf das Gewicht der Reaktionsmasse, verwendet wird, vorzugsweise von 0,1 bis 1 Gew.-%, bezogen auf das Gewicht der Reaktionsmasse, eingesetzt wird.

20. Verfahren nach Anspruch 1,
**dadurch gekennzeichnet, dass** die Vakuum-Destillation von nicht umgesetzter Benzoesäure zusammen mit aromatischem Lösungsmittel bei einer Temperatur von 240 bis 250°C durchgeführt wird.

21. Verfahren nach Anspruch 1,
wobei die Vakuumdestillation-Destillation von nicht umgesetzter Benzoesäure zusammen mit aromatischem Lösungsmittel bei einem Druck von 0,1 bis 30 kPa, vorzugsweise von 2 bis 15 kPa, durchgeführt wird.

22. Verfahren nach Anspruch 1,
wobei zur Benzoesäure das Vorerhitzen der in Schritt (b) destillierten, nicht umgesetzten Benzoesäure, die in Schritt (b) destilliert wurde, gegebenenfalls zusammen mit einem aromatischen Solvent, durchgeführt wird, um die Säurekristalle aufzulösen.

23. Verfahren nach Anspruch 22,
**dadurch gekennzeichnet, dass** das Erhitzen auf die Temperatur erfolgt, bei der die Benzoesäure in die Lösung übergeht, vorzugsweise auf eine Temperatur von 20 bis 150°C, noch bevorzugter von 40 bis 80°C.

24. Verfahren nach Anspruch 1,
wobei die Neutralisation des Benzoesäurerests im rohen Ester mit einer wässrigen Lösung eines alkalischen Mittels, nämlich Natriumhydroxid Natriumcarbonat, Natriumsilikat, Kaliumhydroxid, Calciumcarbonat oder Magnesiumcarbonat ist.

25. Verfahren nach Anspruch 24,
**dadurch gekennzeichnet, dass** die Menge des Alkalimittels 0,5 bis 3 Mol pro Mol der Restsäure, vorzugsweise 1 bis 1,5 Mol pro Mol der Restsäure, beträgt.

26. Verfahren nach Anspruch 1,
**dadurch gekennzeichnet, dass** die Neutralisation bei einer Temperatur von 50 bis 90°C durchgeführt wird.

27. Verfahren nach Anspruch 1,
wobei der Neutralisationsprozess bei atmosphärischem Druck durchgeführt wird.

28. Verfahren nach Anspruch 1,
bei dem die Filtration des Esters bei einer Temperatur von 50 bis 90°C durchgeführt wird.

29. Verfahren nach Anspruch 1,
bei dem die Trocknung des Esters bei einer Temperatur von 105 bis 115°C durchgeführt wird.

## Revendications

1. Méthode de préparation d'esters d'acide benzoïque comprenant les étapes suivantes
a) estérification de l'acide benzoïque et d'un alcool choisi parmi le groupe comprenant les alcools aliphatiques monohydriques ayant un nombre d'atomes de carbone compris entre 6 et 12, ou un alcool polyhydrique ayant un nombre d'atomes de carbone compris entre 2 et 10, en présence d'un catalyseur contenant un metal en présence d'un catalyseur contenant un métal dans un milieu contenant un solvant aromatique, sous la l'augmentation progressive de la température du processus depuis la température de chargement des composants de départ à 180-200°C, puis à 200- 220°C, puis à 240-250°C, afin d'obtenir le mélange réactionnel comprenant l'ester, l'acide benzoïque n'ayant pas réagi et le solvant aromatique ;
b) distillation sous vide de l'acide benzoïque n'ayant pas réagi avec le solvant aromatique du mélange réactionnel obtenu à l'étape (a) pour produire l'ester brut ;
c) recyclage de l'acide benzoïque n'ayant pas réagi distillé à l'étape b) éventuellement avec le solvant aromatique à l'étape d'estérification;
d) neutralisation des résidus d'acide benzoïque dans l'ester brut et lavage à l'eau ;
e) filtration et séchage des esters.

2. Méthode selon la revendication 1,
dans laquelle l'alcool monohydrique aliphatique ayant un nombre d'atomes de carbone compris entre 6 et 12, est hexanol, octanol, dodécanol, 2-éthylhexanol.

3. Méthode selon la revendication 1,
dans laquelle l'alcool polyhydrique ayant de 2 à 10 atomes de carbone, est un alcool diatomique ou un alcool triatomique, de préférence un alcool diatomique est un glycol ayant de 2 à 10 atomes de carbone, en particulier l'éthylène glycol, diéthylène glycol, triéthylène glycol, mono-, di- ou tripropylène glycol, butylène glycol glycol, 2, 2, 4-tri-méthylpentane-1, 3-diol, 1, 2- cyclohexandiméthanol, 1,4-cyclohexandiméthanol, un alcool triatomique est le glycerol plus préférentiellement un alcool diatomique est le glycol ayant de 2 à 10 atomes de carbone, en particulier le diéthylène glycol, le dipropylène glycol et le triéthylène glycol.

4. Méthode selon la revendication 1,
dans laquelle un catalyseur contenant un métal est constitué de composés organiques de titane, d'étain, de zirconium, de préférence un catalyseur contenant un métal est constitué des compositions obtenues par la réaction entre l'ortoester de titane et l'alcool, et de l'alcool, de l'acide et/ou de la base, ou un catalyseur contenant un métal est le titane tétraisopropoxyde de titane ou le tétraisobutoxyde de titane.

5. Méthode selon la revendication 1,
dans laquelle la quantité de catalyseur contenant un métal est de 0,01 à 1 % sur la base de la masse de réaction, de préférence de 0,04 à 0,06% par rapport à la masse de réaction.

6. Méthode selon la revendication 1,
dans laquelle l'estérification de l'acide benzoïque avec un alcool, choisi dans le groupe comprenant les alcools monohydriques ayant un nombre d'atomes de carbone compris entre 6 et 12, ou l'alcool polyhydrique ayant un nombre d'atomes de carbone de 2 à 10, est effectuée dans un rapport molaire acide benzoïque/alcool de 1:1 à 3:1, de préférence de 2:1 à 2,5:1, plus préférentiellement de 2,05:1 à 2,2:1.

7. Méthode selon la revendication 1,
dans lequel le solvant aromatique est un composé carbocyclique dont la molécule a une groupe cyclique à six atomes de carbone appelé benzène ou anneau aromatique.

8. Méthode selon la revendication 7,
dans laquelle ledit composé carbocyclique peut être non substitué, ou peut avoir un ou plusieurs autres composés carbocycliques, les substituants étant des substituants alkyles n'ayant pas plus de 7 atomes de carbone, tels que le méthyle, l'éthyle ou l'isopropyle, et/ou des substituants alcoxy n'ayant pas plus de 7 atomes de carbone, tels que les groupes méthoxy ou éthoxy, de préférence un solvant aromatique est le cumène, le toluène, l'éthylbenzène, le chlorbenzène, tout isomère du xylène (orto-xylène, para-xylène et méthaxylène), mélanges de ceux-ci, plus préférentiellement un solvant aromatique est tout isomère du xylène, ou les mélanges de ceux-ci.

9. Méthode selon la revendication 1,
dans laquelle un solvant aromatique a une température d'ébullition comprise entre 110 et 200°C.

10. Méthode selon la revendication 1,
dans laquelle un solvant aromatique est chargé dans l'étape d'estérification sous forme de solution avec l'acide benzoïque.

11. Méthode selon la revendication 1,
dans laquelle la consommation de solvant aromatique est de 0,01 à 1 l/heure par litre de réaction, de préférence de 0,1 à 0,5 l/heure par litre de masse de réaction.

12. Méthode selon la revendication 1,
dans laquelle un solvant aromatique est ajouté à raison d'au moins 0,05 kg/kg de la masse de réaction, de préférence à raison d'au moins 0,2 kg/kg de la masse de réaction.

13. Méthode selon la revendication 1,
dans laquelle le taux de d'augmentation de la température dans le processus d'estérification est de 0,3 à 0. 5°C/min.

14. Méthode selon la revendication 1,
dans laquelle l'estérification estérification est conduite à une pression de 80 à 150 kPa, de préférence, de 90 à 110 kPa, plus préférentiellement, de 100 à 102 kPa.

15. Méthode selon la revendication 1,
dans laquelle l'estérification est effectuée dans un milieu de gaz inerte.

16. Méthode selon la revendication 1,
dans laquelle l'estérification est réalisée sous agitation intensive.

17. Méthode selon la revendication 1,
dans laquelle un adsorbant est dans l'étape d'estérification.

18. Méthode selon la revendication 17,
dans laquelle l'adsorbant est du charbon actif, du célit, de la perlite.

19. Méthode selon la revendication 17,
dans laquelle l'adsorbant est utilisé à raison de 0,01 à 2 % en poids par rapport au poids de la poids de la masse réactionnelle, de préférence de 0,1 à 1 % en poids par rapport au poids de la masse réactionnelle.

20. Méthode selon la revendication 1,
dans laquelle la distillation sous vide de l'acide benzoïque n'ayant pas réagi avec le solvent aromatique est effectuée à une température comprise entre 240 et 250°C.

21. Méthode selon la revendication 1,
dans laquelle la distillation sous vide de l'acide benzoïque n'ayant pas réagi et du solvant aromatique est effectuée à une pression comprise entre 0,1 et 30 kPa, de préférence entre 2 et 15 kPa.

22. Méthode selon la revendication 1,
dans laquelle, afin de recycler l'acide benzoïque, le chauffage préliminaire de l'acide benzoïque non réagi distillé à l'étape (b), éventuellement avec un aromatique, afin de dissoudre les cristaux d'acide.

23. Méthode selon la revendication 22,
dans laquelle le chauffage est à la température à laquelle l'acide benzoïque passe à la solution, de préférence à la température la solution de 20 à 150°C, plus préférentiellement de 40 à 80°C.

24. Méthode selon la revendication 1,
dans laquelle la neutralisation du résidu d'acide benzoïque dans l'ester brut est effectuée avec une solution aqueuse d'agent alcalin, qui est l'hydroxyde de sodium, le carbonate de sodium, le silicate de sodium, l'hydroxyde de potassium, le carbonate de calcium ou le carbonate de magnésium.

25. Méthode selon la revendication 24,
dans laquelle la quantité d'agent alcalin est de 0,5 à 3 mol par mol de l'acide résiduel, de préférence de 1 à 1,5 mol par mol de l'acide résiduel.

26. Méthode selon la revendication 1,
dans laquelle le processus de neutralisation est conduit à une température de 50 à 90°C.

27. Méthode selon la revendication 1,
dans laquelle le processus de neutralisation est mené à la pression atmosphérique.

28. Procédé selon la revendication 1,
dans lequel la filtration de l'ester est effectuée à une température comprise entre 50 et 90°C.

29. Méthode selon la revendication 1,
dans laquelle le séchage de l'ester est effectué à une température comprise entre 105 et 115°C.
